Europäisches Patentamt

European Patent Office

Office européen des brevets

(11) Publication number: **0 211 258**
A2

## (12) EUROPEAN PATENT APPLICATION

(21) Application number: 86109370.6

(22) Date of filing: 09.07.86

(51) Int. Cl.⁴: **A 61 K 9/10**

(30) Priority: 29.07.85 US 759796

(43) Date of publication of application:
25.02.87 Bulletin 87/9

(84) Designated Contracting States:
AT BE CH DE FR GB IT LI LU NL SE

(71) Applicant: ABBOTT LABORATORIES
14th Street and Sheridan Road North St
North Chicago Illinois 60064(US)

(72) Inventor: Gauthier, Robert Joseph
515 Beverly Place
Lake Forest Illinois 60045(US)

(72) Inventor: Levinson, Robert Saul
4387 Country Trail
Gurnee Illinois 60031(US)

(74) Representative: Modiano, Guido et al,
MODIANO, JOSIF, PISANTY & STAUB Modiano &
Associati Via Meravigli, 16
I-20123 Milan(IT)

(54) Microemulsion compositions.

(57) A composition for parenteral administration is provided.
The composition is a microemulsion consisting essentially of
a discontinuous droplet phase comprising a pharmaceutically
acceptable lipid and/or lipophilic drug and containing from
about 0.6% to about 10% pharmaceutically acceptable emulsifier by weight as the sole emulsifier in the composition, and
having a droplet size such that less than 1% of the droplets
have diameters greater than 0.125 micron (125 nanometers),
in a sterile, non-pyrogenic aqueous continuous phase.

EP 0 211 258 A2

Croydon Printing Company Ltd.

## MICROEMULSION COMPOSITIONS

### Technical Field

This invention relates to emulsion compositions for parenteral administration. More particularly, it relates to emulsions which are commonly called microemulsions, in which the particle size of the disperse phase is typically less than 0.125 micron.

Many materials which are useful in the nutritional or pharmacological therapy of humans and lower animals are insoluble or poorly soluble in water. Since the blood, urine, lymph, cerebrospinal fluid and synovial fluid are all basically aqueous media, such limited solubility impairs the usefulness of such materials for parenteral administration. At the same time, many such materials are readily soluble in lipid, or soluble in lipid preferentially to water. The brain and other nervous tissue, most cell membranes, and many microbial cell walls are rich in lipids, so that these drugs can be surprisingly effective, both as to potency and duration, if delivered intact to their intended sites of action.

The formulation of aqueous dosage forms for such drugs is difficult, if not impossible, due to their insolubility in water and the undesirability of parenteral administration of aqueous suspensions of drugs. In some cases, the poor aqueous solubility of the drug can be overcome by administration as the salt or ester of the pharmacologically active moiety.

However, such modifications to the active compound frequently give rise to other problems. For example, thiopental, which is very lipid-soluble, is commonly administered parenterally as the sodium salt, which is relatively soluble in water. Unfortunately, commonly employed aqueous solutions of thiopental sodium (2.5%, w/v) have a high pH (10.5), which is moderately irritating when administered intravenously, can cause tissue necrosis when injected extravascularly, and can cause disastrous results if accidentally given intraarterially. Other drugs, such as diazepam, are administered in non-aqueous solvents, which are themselves irritating to blood vessels and other tissues, and which produce precipitation of the drug if added to an intravenous solution. Still other drugs are dissolved in water by the use of co-solvents or emulsifiers, which also present their own problems of irritation, insolubility, and toxicity.

In other instances, it is desirable to administer lipids intravenously as a concentrated source of calories. However, in such cases the physical form of the lipid compositions also gives rise to problems. Such materials, especially purified vegetable triglycerides, are commonly administered as emulsions in water, often with added carbohydrates, such as dextrose, added amino acids, or both. Such emulsions contain added emulsifiers to maintain the stability of the emulsion, and are milky white or off-white fluids.

Because the particle size of the disperse, or droplet, lipid phase is as large as or larger that most pathogenic microorganisms, these emulsions cannot be sterilized by filtration - they must be autoclaved. Since added carbohydrates and amino acids would react with one another, or with the triglycerides, under-autoclave conditions, these materials can only be added after the base emulsion has been sterilized, as separate sterile solutions. It would be desirable to have lipid emulsions which could be sterilized by filtration, which does not cause the components to react, so that amino acids and carbohydrates could be added at the manufacturing stage to provide a complete parenteral nutritional formulation in a single package.

In addition, conventional emulsions, particularly if they contain added carbohydrates and amino acids, are fertile breeding grounds for microorganisms. Microbial contamination of transparent solutions can be detected by observing turbidity in the solution when the concentration of microorganisms reaches $10^5$ to $10^6$ per milliliter. However, the opaque, milky appearance of conventional lipid emulsions makes it difficult or impossible to visually detect microbial contamination in the solutions, even at high concentrations of microorganisms. It would be desirable to provide lipids in a relatively tranparent, colorless parenteral dosage form such as the microemulsion which can readily be inspected visually.

A further problem with conventional lipid emulsions for parenteral nutrition is that the particle size of the disperse lipid phase is such that the lipid is not assimilated well if administered rapidly. It has been observed that particles of the size found in the disperse phase of conventional lipid emulsions are removed from the blood stream via the reticuloendothelial cell system (RES) of the body. The RES identifies these particles as foreign and removes them by phagocytosis, a process which is limited by the ability of the RES cells to draw in and break down these large particles. Too-rapid administration of conventional lipid emulsions can overload this process and produce reduced metabolism and lipid toxicity.

Smaller particles can bypass this mechanism and be taken up in some degree directly by a variety of metabolically active cells, resulting in faster assimilation and better utilization by the body.  Thus, it would be desirable to provide a lipid for parenteral nutrition which has a particle size such that it is more readily and more safely assimilated by the body.

It is an objective of this invention to provide a lipid emulsion for parenteral administration which uses minimal amounts of pharmaceutically acceptable emulsifiers.

It is another objective of this invention to provide a lipid emulsion which can serve as a parenteral vehicle for other therapeutically active materials.

A further objective of this invention is to provide a lipid emulsion suitable for opthalmic use.

It is yet another objective of this invention to provide a lipid emulsion for parenteral administration which can be sterilized by filtration.

Still another objective of this invention is to provide a lipid emulsion for parenteral administration or for use as a parenteral vehicle which is substantially clear and colorless.

A further objective of this invention is to provide a lipid emulsion for parenteral nutrition which can be rapidly and safely assimilated by the body.

These and other objectives of this invention will be evident from the following disclosure.

## Background Art

Microemulsions are well known in the scientific arts, and have recently been described for use in pharmaceutical applications.  For example, U.S. Patent 4,340,594, issued July 20, 1982, describes lipid emulsions for parenteral administration having a mean particle size below 1 micron.  However, it must be noted that this emulsification was achieved only by the use of high levels of conventional emulsifiers.

Microemulsification by microfluidization has also recently been described by Chandonnet et al., "Preparation of Microemulsions by Microfluidization", Soap/Cosmetics/Chemical Specialties, February, 1985, the disclosures of which are hereby incorporated herein by reference. However, this article does not address the suitablility of such technology for the preparation of parenteral solutions, and does not suggest the applicability of this technology, along with pharmaceutically safe and acceptable levels of emulsifiers in preparing compositions for injection into the human body.

## Disclosure of the Invention

This invention provides compositions for parenteral administration, consisting essentially of a microemulsion of a discontinuous droplet phase comprising a pharmaceutically acceptable lipid and/or lipophilic drug and containing from about 0.6% to about 10% pharmaceutically acceptable emulsifier by weight as the sole emulsifier in the composition, and having a droplet size such that less than 1% of the droplets have diameters greater than 0.125 micron (125 nanometers), in a sterile, non-pyrogenic aqueous continuous phase.

When used for parenteral nutrition, the compositions of this invention will preferably contain additional nutrients, including micronutrients, as necessary. Therefore, this invention also provides compositions which further contain one or more amino acids or related compounds, electrolytes, carbohydrates, water-soluble vitamins and water soluble micronutrients, including trace elements, and mixtures of any or all of the foregoing.

## Discontinuous Phase

The compositions of this invention have a discontinuous phase component selected from pharmaceutically acceptable lipids, lipophilic drugs, and mixtures thereof. In one class of preferred embodiments of the compositions of this invention, the pharmaceutically acceptable lipid comprises a sterol or a vegetable oil. Sterols include compounds such as cholesterol, chenodeoxycholic acid, ursodeoxycholic acid, and the like. The vegetable oil, if used, is preferably selected from soybean oil triglycerides, safflower oil triglycerides, and blends thereof. Depending upon the desires of the formulator, these triglycerides may desirably comprise a medium chain ($C_6$-$C_{12}$ fatty acid chains) triglyceride fraction, which is known to be more easily metabolized by patients under metabolic or physiologic stress.

Other lipids which can be used in the compositions of this invention include synthetic and semisynthetic mono- di- and/or triglycerides, triglycerides prepared by solvent or thermal fractionation of natural, synthetic or semisynthetic triglycerides, and triglycerides prepared by interesterification and/or directed or random rearrangement.

The compositions of this invention can also be formulated so that the discontinuous phase comprises one or more lipophilic pharmaceutically active agents ("lipophilic drugs"), either alone or dissolved in pharmaceutically acceptable lipid. By "lipophilic" herein is meant agents which are relatively insoluble in water but soluble in one or more of the fat solvents (benzene, chloroform, acetone, ether, hexane, etc.). In particular, the lipid-containing compositions can be used as vehicles for pharmaceutically active agents having lipid component:water partition coefficients of at least 2:1.

Compositions in which the discontinuous phase consists essentially of a lipophilic drug or combination of lipophilic drugs avoid the use of a separate lipid vehicle for the drug, minimize the volume of a unit dose of the drug or drugs, and reduce the risk of toxic effects which might be associated with other vehicles which would otherwise be used.

Lipophilic drugs used in the compositions of this invention are preferably selected from the group consisting of general anesthetics, local anesthetics, hypnotics, sedatives and anxiolytics, antidepressants, anticonvulsants, narcotic analgesics and narcotic antagonists, nonsteroidal antiinflammatory drugs, anticholinesterases, sympathomimetics and parasympathomimetics, ganglionic stimulating and blocking agents, neuromuscular blocking agents, antimuscarinic agents, adrenergic blocking agents, autacoids and autacoid antagonists, digitalis and digitalis congeners, diuretics and saliuretics, antibiotics and antimicrobials, antineoplastics, immunosuppressants and immunomodulators, hemoglobin and hemoglobin derivatives and polymers, hormones and hormone antagonists, and fat-soluble vitamins, and combinations thereof. These terms are used in the sense commonly employed in standard reference texts in the pharmaceutical arts, such as Gilman, Goodman and Gilman, The Pharmacological Basis of Therapeutics, 6th Ed. (MacMillan, New York, 1980), the disclosures of which are hereby incorporated herein by reference.

In particular, the general anesthetics can include, without limitation, diethyl ether, divinyl ether, fluoroxene, methoxyfluorane, halothane, etomidate, anesthetic steroids, enflurane, isoflurane, and combinations thereof.

Similarly, local anesthetics are preferably selected from cocaine, procaine, lidocaine, bupivacaine, chloroprocaine, dibucaine, etidocaine, mepivacaine, prilocaine, cyclomethycaine, hexylcaine and pramoxine, and combinations thereof.

The category of hypnotics, sedatives and anxiolytics includes the barbiturates, such as thiopental and phenobarbital; the benzodiazepines, such as diazepam and chlorazepate; the butyrophenones, such as droperidol and haloperidol; the phenothiazines, such as chlorpromazine and prochlorperazine; the thioxanthines, such as chlorprothixene; as well as the agents loxapine, molindone, chloral hydrate, chloral betaine, ethchlorvynol, ethinamate, glutethimide, meprobamate, methaqualone, methyprylon, paraldehyde, and triclofos; and combinations of any of the foregoing.

An antidepressant can be selected from imipramine, desipramine, amitriptyline, nortriptyline, doxepin, protriptyline, isocarboxazid, phenelzine and tranylcypromine, and combinations these agents.

Anticonvulsants include, without limitation, phenytoin, mephenytoin, ethotoin, primidone, carbamazepine, ethosuximide, methsuximide, phensuximide, valproic acid, valproates, trimethadione, paramethadione, and phenacemide, and combinations thereof.

Many nonsteroidal antiinflammatory drugs are preferentially soluble in lipids, including, without limitation, the salicylates, the vicinal and geminal organophosphonates, the gentisates, phenylbutazone, indomethacin, oxyphenbutazone, antipyrine, aminopyrine, apazone, acetaminophen, phenacetin, sulindac, flufenamates, mefenamates, tolmetin, ibuprofen, naproxen, fenoprofen, flurbiprofen, ketoprofen, colchicine and allopurinol, and combinations of these agents, although most are not commonly used in combination.

Compositions can also be formulated according to this invention wherein the discontinuous lipid phase contains a sympathomimetic selected from the catecholamines, such as epinephrine and isoproterenol; amphetamines, including methamphetamine and hydroxyamphetamine; and the agents ephedrine, mephentermine, metaraminol, phenylephrine, methoxamine, methoxyphenamine, metaproterenol, and terbutaline; and combinations of any of the foregoing.

Adrenergic blocking agents useful in the practice of this invention include the alpha-adrenergic blockers, such as phenoxybanzamine; beta-adrenergic blockers, such as propranolol; adrenergic neuron blockers, such as guanethidine and bretylium; and combinations of these agents.

Autacoids as used herein refers to the natural and synthetic histamines, and 5-hydroxytryptamine, and combinations thereof. Similarly, autacoid antagonists are selected from histamine ($H_1$)-receptor blockers, histamine ($H_2$)-receptor blockers, and 5-hydroxytryptamine antagonists, such as the ergot alkaloids, lysergic acid derivatives, and cyproheptadine, as well as compatible combinations of these compounds.

A variety of diuretic or saliuretic materials can be used in the practice of this invention, including those selected from the carbonic anhydrase inhibitors, such as acetazolamide; the benzothiadiazides, such as hydrochlorothiazide and methyclothiazide; and the agents ethacrynic acid, furosemide, bumetanide, muzolimine, spironolactone, triamterene, amiloride, ticrynafen and indacrynic acid, and combinations thereof.

Antimicrobial, antiviral or antibiotic compounds useful in the practice of this invention include the penicillins, whether natural or synthetic; erythromycins; sulfonamides; cephalosporins; aminoglycosides; tetracyclines; rifamycins; lincomycins and polymyxins, as well as chloramphenicol, isoniazid, pyrazinamide, cycloserine, viomycin, clindamycin, spectinomycin, vancomycin, nystatin, amphotericin B, flucytosine, griseofulvin, amantadine, methisazone, vidarabine, idoxuridine, acyclovir and interferon, and combinations thereof.

Antineoplastic agents include compounds from a variety of classes, such as ethyleneimine derivatives, alkyl sulfonates, nitrosoureas, triazenes, folic acid analogs, pyrimidine analogs, purine analogs, vinca alkaloids, l-asparaginase, dactinomycin, daunorubicin, doxorubicin, bleomycin, mithramycin, mitomycin, platinum coordination complexes, substituted ureas, procarbazine, mitotane and tamoxifen, and combinations thereof.

The hormones include growth hormone, prolactin, placental lactogen, luteinizing hormone, follicle stimulating hormone, thyrotropin, chorionic gonadotropin, chorionic thyrotropin, corticotropin, alpha- and beta-melanocyte stimulating hormones, beta- and gamma-lipotropins, endorphins, enkephalins, estrogens, progestins, androgens and anabolic steroids, glucocorticoids and glucocorticoid derivatives, mineralocorticoids and mineralocorticoid derivatives, insulin, glucagon, parathyroid hormone, thyroid hormone, prostaglandins, calcitriol and calcitonin. Many hormones and synthetic derivatives are readily lipid soluble, particularly the synthetic steroid hormones.



Combinations of the foregoing hormones can also be used where appropriate.

Cyclosporin is a prime example of the immunomodulators. This is a relatively new class of drugs, and few of these compounds have been approved for clinical use. Nevertheless, the use of lipid-soluble immunomodulators in the compositions of this invention is fully contemplated.

Hemoglobin and hemoglobin derivatives and polymers includes human, animal or synthetic hemoglobin per se as well as derivatives of human, animal or synthetic hemoglobin. Derivatives include hemoglobin oligomers and polymers, liposomal hemoglobin, and hemoglobin linked to other carrier or active compounds or polymers, such as hemoglobin linked to 2-nor-2-formylpyridoxal 5'-phosphate.

The fat-soluble vitamins overlap some of the foregoing categories to a certain extent. As examples, Vitamin E is a tocopherol antioxidant, and Vitamin D (calciferol) is classified as a hormone. The fat soluble vitamins also encompass the carotenes, including Vitamin A and Vitamin A precursors; synthetic calciferols and tocopherols; Vitamin K, including the menaquinones, phytonadione and menadione; and combinations thereof.

In these preferred compositions, the emulsifier comprises nontoxic phospholipids. Most preferably, the emulsifier comprises egg phosphatides, which are commonly used in the pharmaceutical arts.

Because of the high particle surface area offered by the particulate products of this invention, the ingredients may be prone to oxidation, particularly if the content of unsaturated fatty acid moieties is high. In such cases, the compositions will preferably also contain a tocopherol or other lipophilic antioxidant.

## Aqueous Continuous Phase

The aqueous continuous phase can, if desired, comprise a solute selected from amino acids and related compounds, electrolytes, water-soluble vitamins, water-soluble trace elements, and carbohydrates, and mixtures thereof. The aqueous continuous phase can also contain parenteral adjuvant materials, such as those used to adjust tonicity and pH.

The amino acids administered in the compositions of this invention can include, without limitation, leucine, isoleucine, lysine, methionine, phenylalanine, threonine, tryptophan, valine, alanine, arginine, histidine, proline, serine, tyrosine, glycine, taurine and carnitine, as the L-, D-, or racemic forms, as well as nontoxic salts and esters thereof. Most preferred are the L-acids, and their nontoxic salts and esters. Of particular value in formulating the compositions of this invention is the amino acid carnitine, which has been reported to facilitate the metabolism of lipids and other amino acids. When added to the compositions of this invention, the carnitine is preferably added in an amount sufficient to enhance metabolism of the lipids in the discontinuous phase, but can be added in am amount sufficient to enhance the metabolism of other amino acids in the continuous phase.

The amino acids added to the compositions of this invention also preferably comprise branched chain amino acids, which are reported to facilitate normalization of plasma amino acid levels, particularly in debilitated or liver-damaged patients.

Compounds related to the amino acids include the keto- analogs of all of the above-mentioned amino acids, partial hydrolysates of protein, and oligo- or polypeptides of synthetic origin.

According to the desires of the formulator, electrolytes added to the compositions of this invention can include, without limitation, sodium, potassium, magnesium, calcium, lithium, ammonium, phosphorus, chloride, iodide, bromide, fluoride, acetate, sulfate, carbonate, phosphate, lactate, gluconate and lactobionate in pharmaceutically acceptable amounts. In measuring or calculating the electrolyte content of the aqueous phase, the electrolyte equivalents of any amino acids and amino acid salts included in the formulation should also be taken into consideration.

The water-soluble vitamins useful in the compositions of this invention are well known and include the B-vitamins, Vitamin C, and minor vitamins such as bioflavonoids and biotin. The water-soluble trace elements include chromium, selenium, copper, calcium, zinc, magnesium and iron.

Carbohydrates useful in formulating the compositions of this invention include monosaccharides, disaccharides, oligosaccharides, and polysaccharides. The monosaccharides as defined herein include, without limitation, the sugars dextrose, fructose and galactose, and the sugar alcohols mannitol, xylitol, inositol and sorbitol. The disaccharides include, without limitation, sucrose, lactose and maltose. Oligosaccharides include polymers of the above-described monosaccharide sugars containing from 3 to 6 units. Polysaccharides include the well-known dextrans, typically having molecular weights of from 40,000 to 70,000, and such materials as hydroxyethyl starch.

"Pharmaceutically acceptable" is used herein to refer to those materials which are, within the scope of sound medical judgment, suitable for use in contact with the tissues of humans and lower animals without undue toxicity, irritation, allergic response, and the like, commensurate with a reasonable benefit/risk ratio, and effective for their intended use in the composition.

## Physical Properties

Although they contain only the concentrations of phospholipid specified above as the sole emulsifier, the compositions of this invention are relatively stable upon storage. Because of the small particle size of the disperse phase, these microemulsions can be sterilized by submicron filtration.

Compositions can also be formulated according to this invention which are substantially transparent, i.e., have a turbidity of less than 30 Nephelometric Turbidity Units (NTU) when measured by standard published techniques. In some instances, the addition of optional ingredients as provided herein (e.g., carotenes in the lipid phase or riboflavin in the aqueous phase) will impart a color or haze to the composition.

## Dosage Forms

The compositions of this invention are formulated for parenteral administration. By parenteral administration is meant routes of administration other than enteral and topical, usually by injection, and includes, without limitation, intravenous, intraarterial, intrathecal, perineural, intracardiac, intraperitoneal, transtracheal, intramuscular, subcutaneous, subcuticular, intraarticular, subcapsular, subarachnoid, and epidural injection.

Dosage forms for parenteral administration are usually divided into two categories: large volume parenteral (LVP) dosage forms and small volume parenteral (SVP) dosage forms. LVP dosage forms are commonly administered directly by the intravenous route when a large volume of fluid is required or desired to be given, while small volume parenterals are commonly administered in single or multiple injections via any of the above-mentioned routes. Alternatively, a drug provided in SVP dosage form is added to a LVP dosage form to provide diluted, but continuous, administration of the drug.

LVP dosage forms are commonly provided in volumes of from 100 ml (which is considered a LVP dose for pediatric use) to 3000 ml. These volumes of fluid are available both in glass bottles and rigid, semi-rigid, and flexible containers fabricated from a variety of polymers and polymer combinations well known to the art. Each of these forms is suitable for use in the practice of the present invention, provided the usual standards for premarketing regulatory approval of parenteral solution containers are met.

SVP dosage forms are available in greater variety, depending upon the specific drug involved, its intended use, and the volume of fluid to be administered. SVP dosage forms typically contain from 0.1 ml to 100 ml of fluid. These dosage forms include glass and polymeric ampuls; glass and polymeric "fliptop" vials, which are intended for use in filling syringes; pressurized and unpressurized "pintop" vials, which incorporate a transfer cannula and are intended for use in adding the contents to LVP dosage forms; and a number of styles of prefilled syringes. Each is suitable for use in the practice of this invention, subject only to the usual criteria which dictate selection of such dosage forms.

## Preparation

In emulsification, the form of mechanical energy input defines the interfacial region where movement of surfactant between layers of disperse phase creates smaller and smaller droplets. Unlike conventional microemulsion preparations, which require use of high levels of emulsifier, the emulsions of this invention use emulsifier levels usually associated with conventional parenteral lipid emulsions and achieve microemulsification by using a formation (mechanical energy input) process which focuses the work input on droplet size reduction via microfluidization. Equipment for this purpose is commercially available from Microfluidics Corporation, Newton, Mass., but was previously unapplied to medical emulsions. This droplet formation involves both laminar and turbulent flow, as well as cavitation. Two streams interact in precisely defined microchannels at high pressures. This static technique exposes each volume of fluid to forces which are relatively consistent over the entire pass. This produces droplet sizes below 100 nanometers with a narrow size distribution. Increasing the number of passes through the device further decreases the particle size and narrows the particle size distribution.

## Industrial Applicability

The practice of this invention and its industrial applicability can be further understood by reference to the following Examples. These Examples are intended to be illustrative of this invention, and not limitative thereof.

## Example 1

1.2 gram of purified egg phosphatides was dispersed in 75.0 ml of sterile distilled water containing 2.5 grams of glycerin, USP using a Branson ultrasonic probe. After dispersion was effected, the phosphatide/glycerin/water mixture was transferred to the feed vessel of a Microfluidics Microfluidizer, Model 110A. The Microfluidizer was operated at an internal interaction chamber pressure of from 16,500 psi and 19,000 psi. 10.0 grams of soybean oil were slowly injected into the inlet stream of the Microfluidizer as the phosphatide/glycerin/water mixture was being introduced into the unit. The mixture was processed for at least 16 discrete passes through the unit, and the resulting volume of fluid was increased to 100.0 ml using sterile distilled water. This volume was passed through the unit again to insure uniformity of mixture, and the fluid was removed for measurement.

The particle size of the discontinuous phase of a sample of the fluid was measured using a commercial Nicomp laser light scattering instrument and the average particle diameter was found to be less than 100 nanometers (0.1 micrometer). The remainder of the preparation was divided into two parts. One part was sterilized by filtration through a 0.22 micrometer membrane filter without emulsion separation or creaming. The other was sterilized by autoclaving at 121°C at 15 psi for 20 minutes.

### Example 2

1.2 gram of purified egg phosphatides and 50.0 mg of erythromycin base, USP were dispersed in 75.0 ml of sterile distilled water using a Branson ultrasonic probe. After crude dispersion was effected, the mixture was made up to 100.0 ml volume with sterile distilled water. The resulting material was transferred to the feed vessel of a Microfluidics Microfluidizer device, Model 110A. The Microfluidizer was operated at an internal interaction chamber pressure of from 16,500 psi and 19,000 psi. The mixture was processed for at least 16 discrete passes through the unit. The resulting fluid was removed for measurement.

The particle size of the discontinuous phase of a sample of the fluid was measured using a commercial Nicomp laser light scattering instrument and the average particle diameter was found to be less than 100 nanometers (0.1 micrometer). The remainder of the preparation was divided into two parts. One part was sterilized by filtration through a 0.22 micrometer membrane filter without emulsion separation or creaming. The other was sterilized by autoclaving at 121°C at 15 psi for 20 minutes.

### Example 3

1.2 gram of purified egg phosphatides and 50.0 mg of erythromycin base, USP were dispersed in 75.0 ml of sterile distilled water using a Branson ultrasonic probe. After crude dispersion was effected, the mixture was transferred to the feed vessel of a Microfluidics Microfluidizer device, Model 110A. The Microfluidizer was operated at an internal interaction chamber pressure of from 16,500 psi and 19,000 psi. Between 1.0 and 10.0 ml of soybean oil was slowly injected into the inlet stream of the Microfluidizer as the phosphatide/glycerin/water mixture was being introduced into the unit. The mixture was processed for at least 16 discrete passes through the unit, and the resulting volume of fluid was increased to 100.0 ml using sterile distilled water. This volume was passed through the unit again to insure uniformity of mixture, and the resulting fluid was removed for measurement.

The particle size of the discontinuous phase of a sample of the fluid was measured using a commercial Nicomp laser light scattering instrument and the average particle diameter was found to be less than 100 nanometers (0.1 micrometer). The remainder of the preparation was divided into two parts. One part was sterilized by filtration through a 0.22 micrometer membrane filter without emulsion separation or creaming. The other was sterilized by autoclaving at 121°C at 15 psi for 20 minutes.

CLAIMS:

1. A composition for parenteral administration, consisting essentially of a microemulsion of a discontinuous phase component selected from pharmaceutically acceptable lipids, lipophilic drugs, and mixtures thereof and containing from about 0.6% to about 10% phospholipid emulsifier by weight as the sole emulsifier in the composition and having a droplet size such that less than 1% of the droplets have diameters greater than 0.125 microns, in a sterile, non-pyrogenic aqueous continuous phase.

2. A composition according to Claim 1 wherein the pharmaceutically acceptable lipid comprises a vegetable oil.

3. A composition according to Claim 4 wherein the vegetable oil further comprises a medium chain triglyceride fraction.

4. A composition according to Claim 6 wherein the phospholipid emulsifier comprises egg phosphatides.

5. A composition according to Claim 1 which further comprises a solute component selected from amino acids, carbohydrates, electrolytes, water-soluble vitamins and water-soluble trace elements, and mixtures thereof.

6. A composition according to Claim 10 wherein the amino acids comprise branched chain amino acids.

7. A composition according to Claim 1 wherein the discontinuous phase comprises a lipophilic drug.

8. A composition according to Claim 14 wherein the lipophilic drug has a discontinuous phase:continuous phase partition coefficient of at least 2:1.

9.  A composition according to Claim 14 wherein the lipophilic drug is selected from the group consisting of general anesthetics, local anesthetics, hypnotics, sedatives and anxiolytics, antidepressants, anticonvulsants, narcotic analgesics and narcotic antagonists, nonsteroidal antiinflammatory drugs, anticholinesterases, sympathomimetics and parasympathomimetics, ganglionic stimulating and blocking agents, neuromuscular blocking agents, antimuscarinic agents, adrenergic blocking agents, autacoids and autacoid antagonists, digitalis and digitalis congeners, diuretics and saliuretics, antibiotics and antimicrobials, antineoplastics, immunosuppressants and immunomodulators, hemoglobin and hemoglobin derivatives and polymers thereof, hormones and hormone antagonists, and fat-soluble vitamins, and combinations thereof.